# EUROPÄISCHE PATENTSCHRIFT

(11) **EP 0 891 369 B1**
(45) Veröffentlichungstag und Bekanntmachung des Hinweises auf die Patenterteilung: **16.08.2001**
(21) Anmeldenummer: 97920510.1
(22) Anmeldetag: 14.03.1997
(51) Int. Cl.: C07H 1/08, C12Q 1/68, C12N 15/10, C12M 1/00

(54) **VERFAHREN UND VORRICHTUNG ZUR ISOLIERUNG VON NUKLEINSÄUREN**
PROCESS AND DEVICE FOR ISOLATING NUCLEIC ACIDS
PROCEDE ET DISPOSITIF POUR ISOLER DES ACIDES NUCLEIQUES

(30) Priorität: 15.03.1996 DE 19610354
(43) Veröffentlichungstag der Anmeldung: 20.01.1999
(73) Patentinhaber: Inova Gesellschaft zur Entwicklung und Vermarktung Innovativer Produkte mbH, 68307 Mannheim (DE)
(72) Erfinder: LANGE, Hans, D-68623 Lampertheim (DE)
(74) Vertreter: Gassner, Wolfgang, Dr.
(86) Internationale Anmeldenummer: DE9700517
(87) Internationale Veröffentlichungsnummer: WO9734908

(56) Entgegenhaltungen:
- EP-A- 0 687 502
- DE-A- 4 139 664
- FR-A- 2 402 716
- US-A- 5 340 449

## Beschreibung

Die Erfindung betrifft ein Verfahren und eine Vorrichtung zur Isolierung von Nukleinsäuren.

Vor der Analyse von aus Zellen gewonnenen Nukleinsäuren mittels Polymerasekettenreaktion (PCR) ist es erforderlich, die Nukleinsäuren aufzureinigen und aufzukonzentrieren. Ferner kann es notwendig sein, aus der zu analysierenden Probe bestimmte die Polymerasekettenraktion störende Substanzen, wie die prosthetische Gruppe von Hämoglobin, von der Probe abzutrennen.

Daneben spielt auch bei anderen Techniken zur Analyse von Nukleinsäuren, bsp. der Hybridisierung, eine Aufreinigung und Aufkonzentration der zu analysierenden Nukleinsäuren eine wichtige Rolle.

Aus "Methods of Enzymology", Vol. 68, S. 170 - 182, ist es bekannt, zur Isolierung von Nukleinsäuren sog. "Spin-columns" zu verwenden. Dabei werden in einer aus der DE 41 39 664 Al bekannten Variante folgende Arbeitsschritte verwendet:
aa) Zellaufschluß,
bb) Adsorption der Nukleinsäuren an einem Glasfaservlies in Gegenwart eines Puffers mit hoher Ionenstärke und
cc) Elution der Nukleinsäuren mit einem Puffer geringer Ionenstärke.

Beim Schritt lit.aa) wird die flüssige Probe durch ein Glasfaservlies geleitet, an dem die Nukleinsäuren adsorbieren. Anschließend wird das Glasfaservlies mit verschiedenen Lösungen gewaschen. Schließlich werden die Nukleinsäuren in Anwesenheit von Puffern geringer lonenstärke von der Festphase eluiert.

Das bekannte Verfahren ist in mehrfacher Hinsicht nachteilig: Beim Waschen der Festphase kann es zur Kontamination der Probe kommen. Außerdem können wegen der im Glasfaservlies herrschenden Kapillarkräfte die Nukleinsäuren nur zum Teil daraus zurückgewonnen werden.

Des weiteren sind aus "Methods in Enzymology 65" (1980), S. 371 - 380 gelelektrophoretische Methoden bekannt, bei denen Nukleinsäuren an Gele gebunden und danach mittels Elektroelution wieder in Lösung gebracht werden. Auch dabei kann es zur Kontamination der Lösung kommen. Die Nukleinsäuren liegen in der Lösung in hoher Verdünnung vor. Eine Aufkonzentration findet nicht statt.

Aus der FR A 2 402 716 sind ein Verfahren und eine Vorrichtung bekannt, bei denen geladene Makromoleküle mittels Affinitätschromatograhpie an einem Adsorptionsmittel gebunden und anschließend von dort durch Elektrophorese eluiert werden.

Aufgabe der vorliegenden Erfindung ist es, ein Verfahren und eine Vorrichtung anzugeben, mit denen die Nachteile des Stands der Technik vermieden werden. Insbesondere soll ein Verfahren und eine Vorrichtung zur Isolierung von Nukleinsäuren angegeben werden, die eine einfache und kostengünstige Aufreinigung und Aufkonzentration von Nukleinsäuren ermöglichen. Außerdem soll eine weitgehend automatisierte Isolation und Aufkonzentration von Nukleinsäuren durchführbar sein. Schließlich bezweckt die Erfindung die Vermeidung von Kontaminationen.

Diese Aufgabe wird durch die Merkmale der Ansprüche 1 und 12 gelöst. Zweckmäßige Ausgestaltungen des Verfahrens bzw. der Vorrichtung ergeben sich aus den Merkmalen der Ansprüche 2 bis 11 sowie 13 bis 41.

Nach Maßgabe der Erfindung ist ein Verfahren zur Isolierung von Nukleinsäuren aus biologischen nukleinsäurehaltigen Flüssigkeiten und Suspensionen vorgesehen, wobei
a) die Nukleinsäuren an ein Adsorptionsmittel gebunden werden,
b) die Nukleinsäuren vom Adsorptionsmittel eluiert und
c) durch Elektrophorese von einem Reaktionsraum in einen damit verbundenen Entnahmeraum bewegt und dort angereichert werden.

Das Verfahren ermöglicht auf einfachem Weg eine Aufreinigung und Aufkonzentration von Nukleinsäuren aus Flüssigkeiten. Insbesondere bei einer automatischen Verfahrensführung kann das Risiko einer Kontamination weitgehend ausgeschlossen werden. Die Elution kann durch Pufferwechsel oder elektrisch durch Elektroelution bzw. Elektrophorese erfolgen.

Nach weiterer Maßgabe der Erfindung ist eine Vorrichtung zur Isolierung von Nukleinsäuren aus biologischennukleinsäurehaltigen Flüssigkeiten und Suspensionen vorgesehen, wobei ein Reaktionsraum zur Aufnahme eines mit Nukleinsäuren beladenen Adsorptionsmittels mit einem Entnahmeraum verbunden ist, und wobei die Nukleinsäuren mittels einer Elektrophoreseeinrichtung vom Reaktions- in den Entnahmeraum bewegbar und dort anreicherbar sind. - Diese Vorrichtung ermöglicht eine einfach und kostengünstig durchführbare Aufkonzentration und Isolierung von Nukleinsäuren. Durch das Vorsehen eines besonderen Entnahmeraums kann eine Kontamination weitgehend ausgeschlossen werden.

Nachfolgend werden Ausführungsbeispiele der Erfindung anhand der Zeichnung näher erläutert. Hier zeigen:
- Fig. 1: eine schematische Querschnittsansicht eines ersten Ausführungsbeispiels der Vorrichtung,
- Fig. 2: das Ausführungsbeispiel gemäß Fig. 1 mit "Spin-Column",
- Fig. 3: eine schematische Querschnittsansicht eines zweiten Ausführungsbeispiels der Vorrichtung,
- Fig. 4: eine schematische Querschnittsansicht durch ein erstes Ausführungsbeispiel einer Aufreinigungs- und Anreicherungsvorrichtung mit einer Vorrichtung gemäß Fig. 3,
- Fig. 5: eine Draufsicht auf ein Agaroseflachbettgel,
- Fig. 6: eine schematische Querschnittsansicht durch eine beschichtete Elektrode,
- Fig. 7: eine schematische Querschnittsansicht durch ein Ausführungsbeispiel der Vorrichtung,
- Fig. 8: eine Draufsicht auf ein Ausführungsbeispiel einer Elutionsvorrichtung und
- Fig. 9: eine schematische Querschnittsansicht durch ein zweites Ausführungsbeispiel einer Aufreinigungs- und Anreicherungsvorrichtung.

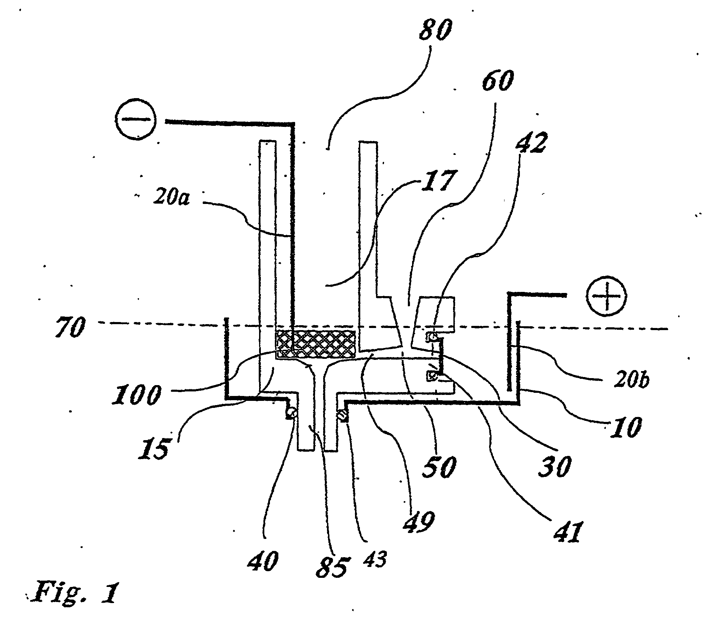

In Fig. 1 ist ein schematischer Querschnitt eines ersten Ausführungsbeispiels der Vorrichtung gezeigt. In einem Elektrophoresepuffertank 10 mit einem bodenseitigen Durchbruch 43 ist ein Behälter 15 aufgenommen. Der Behälter 15 umschließt einen Reaktionsraum 17, welcher über einen Kanal 49 mit einem Entnahmeraum 50 verbunden ist. Das Volumen des Reaktionsraums 17 beträgt vorzugsweise 1 bis 20 ml. Der Entnahmeraum 50 ist mittels einer eine erste Öffnung 42 verschließenden ersten permeablen Membran 30 ionenleitend mit einem im Elektrophoresepuffertank 10 aufgenommenen Elektrophoresepuffer verbunden. Das Volumen des Entnahmeraums 50 beträgt vorzugsweise 0,005 bis 0,1 ml. Die erste permeable Membran 30 ist z.B. aus einer Dialysemembran gebildet, welche für Nukleinsäuren nicht durchlässig, für Salze, insbesondere chaotrope Salze, jedoch durchlässig ist. Die erste permeable Membran 30 ist mittels eines flexiblen Rings, bsp. eines O-Rings, auf einem ersten Stutzen 41 befestigt. Im Reaktionsraum 17 ist ein Adsorptionsmittel 100, bsp. ein Glasfaservlies, Silika, Glasperlen, mit Glas umfangene magnetische Partikel, Anionenaustauscher o. dgl., aufgenommen. In den Reaktionsraum 17 ragt durch eine zweite Öffnung 80 eine Kathode 20a. Eine Anode 20b taucht in den im Elektrophoresepuffertank 10 befindlichen Elektrophoresepuffer ein, dessen Füllstand mit 70 bezeichnet ist. Unterhalb des Adsorptionsmittels 100 erstreckt sich vom Behälter 15 ein zweiter Stutzen 85, der den Durchbruch 43 durchgreift. Der zweite Stutzen 85 ist mittels eines 0-Rings 40 gegenüber dem Elektrophoresepuffertank 10 abgedichtet. - Der Entnahmeraum 50 weist eine Entnahmeöffnung 60 auf. Er ist so ausgebildet, daß Lufteinschlüsse vermieden werden. Der Entnahmeraum 50 kann insbesondere als Kapillare ausgebildet sein.

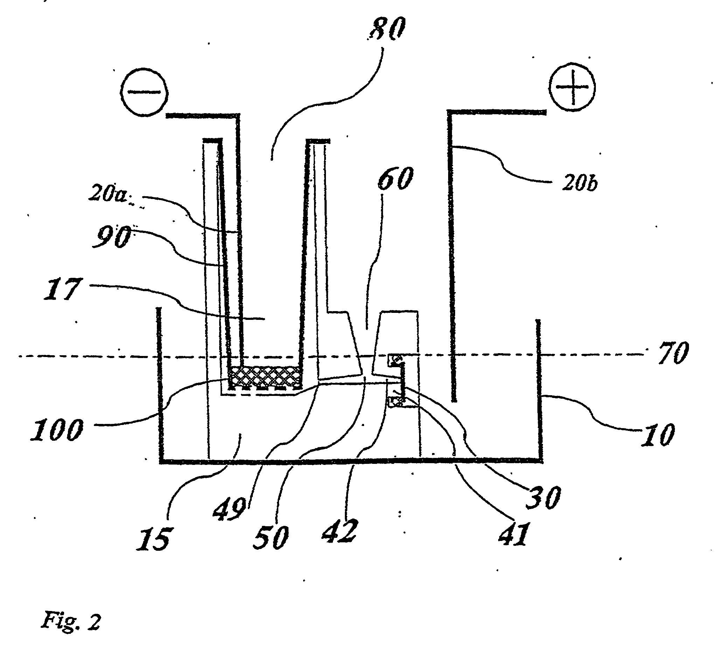

Fig. 2 zeigt im wesentlichen das in Fig. 1 gezeigte erste Ausführungsbeispiel. Dabei ist im Reaktionsraum 17 ein "Spin-column" 90 aufgenommen. Ein den Elektrophoresepuffertank 10 durchgreifender zweiter Stutzen 85 ist hier nicht vorgesehen.

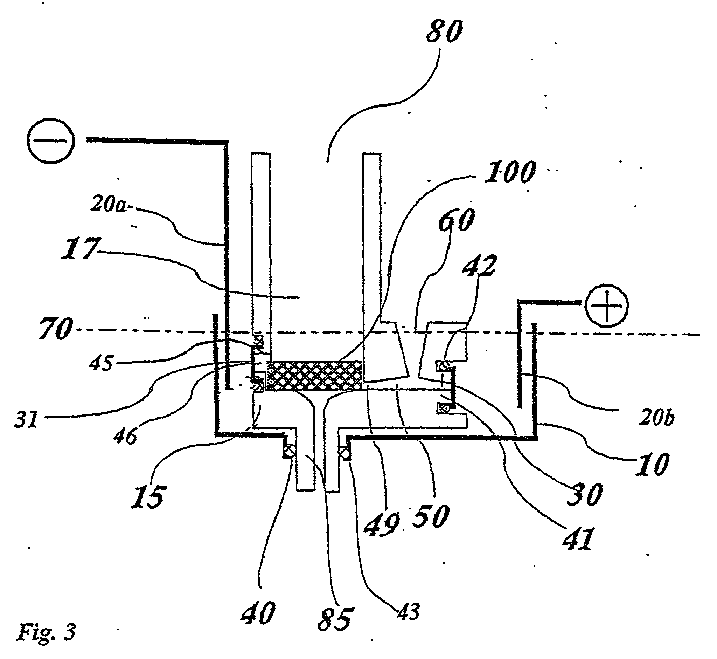

In Fig. 3 ist eine schematische Querschnittsansicht durch ein zweites Ausführungsbeispiel der Vorrichtung gezeigt. Dabei befindet sich die Kathode 20a außerhalb des Reaktionsraums 17. Sie taucht direkt in den Elektrophoresepuffertank 10 ein. Am den Reaktionsraum 17 umgreifenden Teil des Behälters 15 ist ein dritter Stutzen 45 vorgesehen, dessen dritte Öffnung 46 durch eine zweite permeable Membran 31 verschlossen ist.

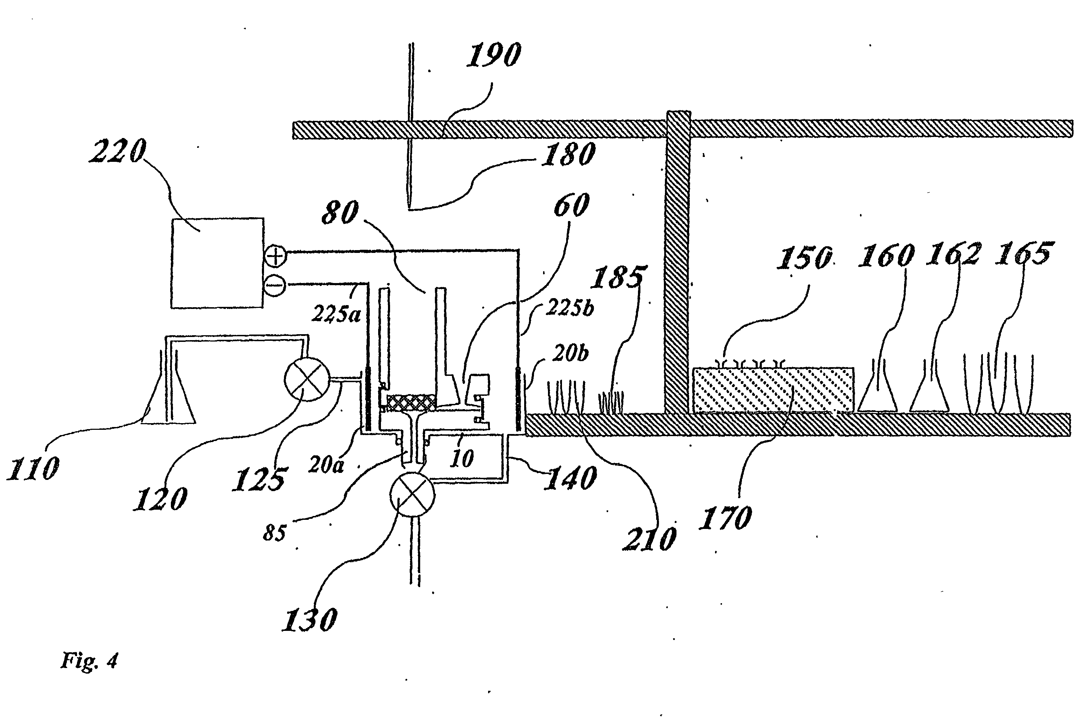

Fig. 4 zeigt eine schematische Querschnittsansicht durch ein erstes Ausführungsbeispiel einer Aufreinigungs- und Anreicherungsvorrichtung mit der Vorrichtung gemäß Fig. 3. Die Vorrichtung gemäß Fig. 3 befindet sich im Wirkbereich eines x, y,z-Pipettors, dessen x,y,z-Pipettierarm mit 190 bezeichnet ist. Der x,y,z-Pipettierarm 190 nimmt eine Pipettierspitze 180, vorzugsweise eine Wegwerfspitze, auf. Ein geeigneter x,y,z-Pipettor wird bsp. von der Firma TECAN AG, Schweiz, angeboten. Ferner ist im Wirkbereich des x,y,z-Pipettors ein heizbarer Schüttelbock 170 angeordnet. Im Schüttelbock 170 sind Reaktionsröhrchen 150 für die Lyse aufgenommen. Daneben befinden sich ein erstes Gefäß 160 für die Lyse, ein zweites Gefäß 162 zur Aufnahme für eine Waschlösung sowie Probengefäße 165. Mit 185 ist ein Vorrat an Pipettenspitzen und mit 210 sind PCR-Gefäße bezeichnet.

Der Elektrophoresepuffertank 10 ist mit einem Füllstutzen 125 versehen, der mit einem Elektrophoresepuffervorrat 110 unter Zwischenschaltung einer Pumpe 120 verbunden ist. Der zweite Stutzen 85 sowie eine am Boden des Elektrophoresepuffertanks 10 vorgesehene Ableitung 140 stehen mit einer zweiten Pumpe 130 in Verbindung. Die zweite Pumpe 130 ist vorzugsweise als Schlauchpumpe ausgeführt. Eine geeignete Schlauchpumpe wird bsp. von der Firma Cavro, Kalifornien, USA, angeboten. Die Kathode 20a sowie die Anode 20b sind über elektrische Zuleitungen 225a bzw. 225b mit einer Spannungsquelle 220 verbunden. Die erste 120 und die zweite Pumpe 130, der Schüttelbock 170, der x,y,z-Pipettor sowie die Spannungsquelle 220 sind so ausgeführt, daß sie mittels eines Prozeßrechners ansteuerbar sind. Somit ist ein vollautomatischer Betrieb der Aufreinigungs- und Anreicherungsvorrichtung möglich.

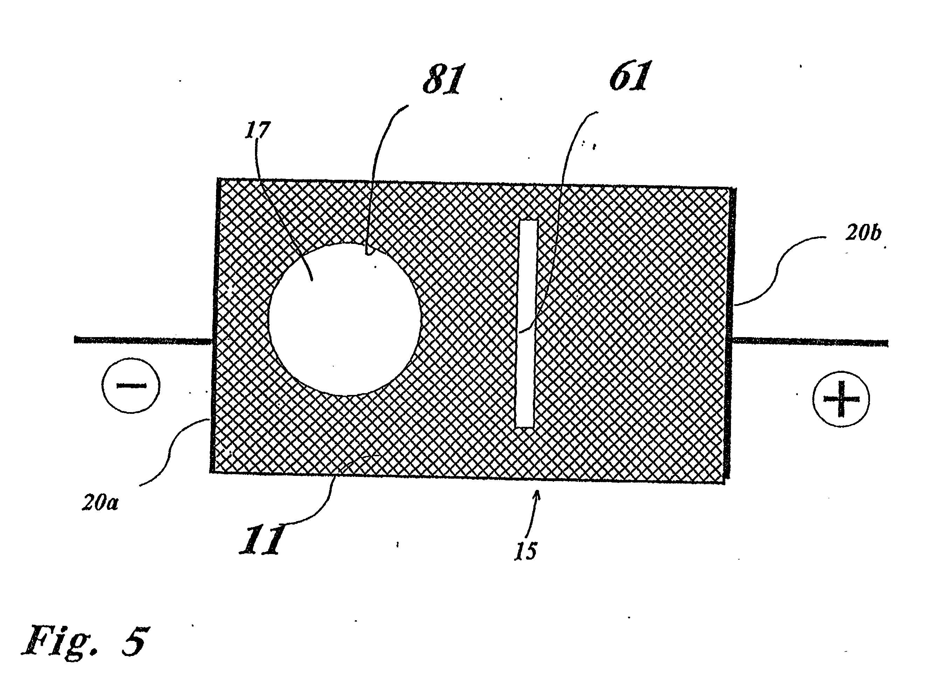

In Fig. 5 ist eine Draufsicht auf eine besonders einfache Variante eines Behälters 15 gezeigt. Dieser ist aus einem Agaroseflachbettgel 11 hergestellt, an dessen gegenüberliegenden Querseiten die Kathode 20a und die Anode 20b anliegen. Im Agaroseflachbettgel 11 ist eine erste den Reaktionsraum 17 bildende Ausnehmung 81 zur Aufnahme von Adsoptionsmittel und eine zweite Ausnehmung 61 vorgesehen. Die zweite Ausnehmung 61 ist schlitzförmig ausgebildet. Sie dient zur Entnahme der darin angereicherten Nukleinsäuren.

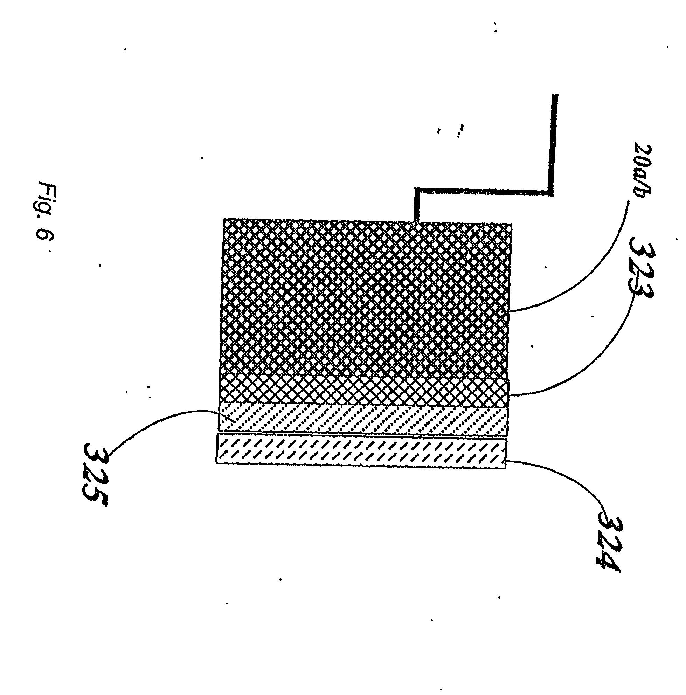

Fig. 6 zeigt eine schematische Querschnittsansicht durch eine beschichtete Elektrode. Eine aus einem Edelmetall, wie Gold, Silber oder Platin, oder elektrisch leitfähigem Kunststoff hergestellte Kathode oder Anode 20a bzw. 20b ist mit einer aus mehreren Lagen bestehenden Beschichtung versehen. Eine erste 323 auf das Edelmetall oder den Kunststoff aufgebrachte Lage besteht aus biotinyliertem Rinderserum Albumin, eine darauf auflagernde zweite Lage 325 besteht aus einem Streptavidin oder Polystreptavidin und eine äußere dritte Lage 324 ist aus einem Oligonukleotid gebildet.

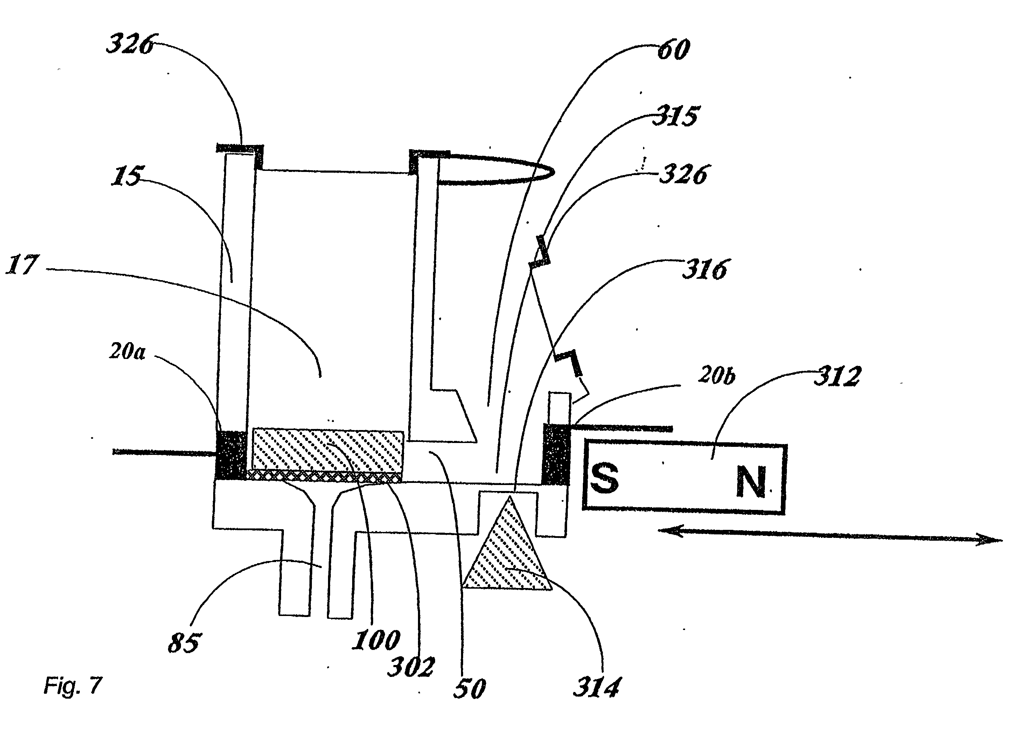

In Fig. 7 ist ein schematischer Querschnitt eines Ausführungsbeispiels gezeigt. Hier ist der bewegliche Permanentmagnet 312 mit seinem Südpol in der Nähe der Außenseite der Anode 20b angeordnet. Der Boden des Entnahmeraums 50 ist durchsichtig. Gegenüber der Entnahmeöffnung 60 befindet sich unterhalb des Bodens des Entnahmeraums 50 der Photomultiplier 314.

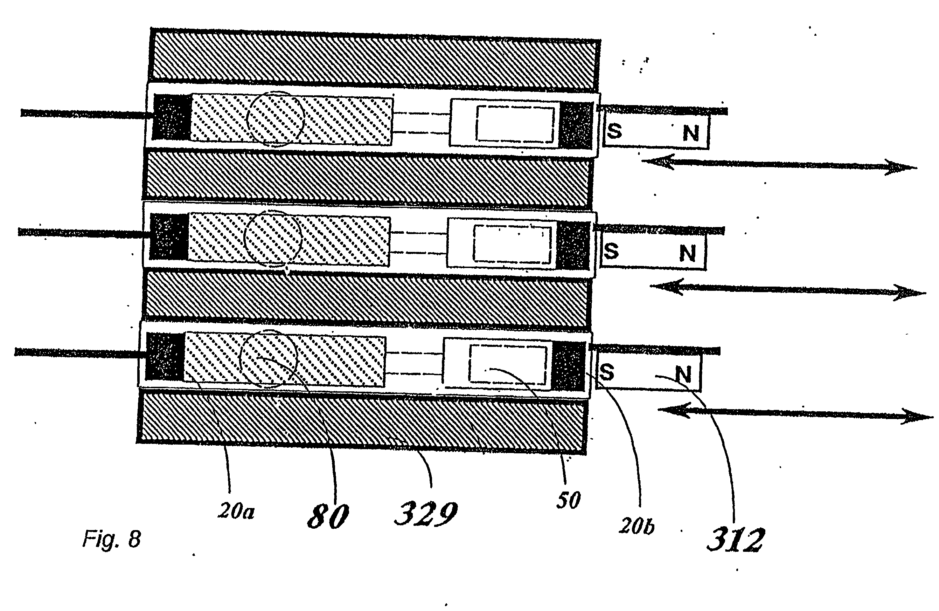

Fig. 8 zeigt eine Draufsicht auf ein Ausführungsbeispiel einer Elutionsvorrichtung. Dabei sind eine Mehrzahl der in Fig. 7 gezeigten Vorrichtungen nebeneinander angeordnet. An den Längsseiten der Vorrichtungen sind jeweils Thermostatplatten 329 vorgesehen, mit denen die Temperatur einstellbar ist.

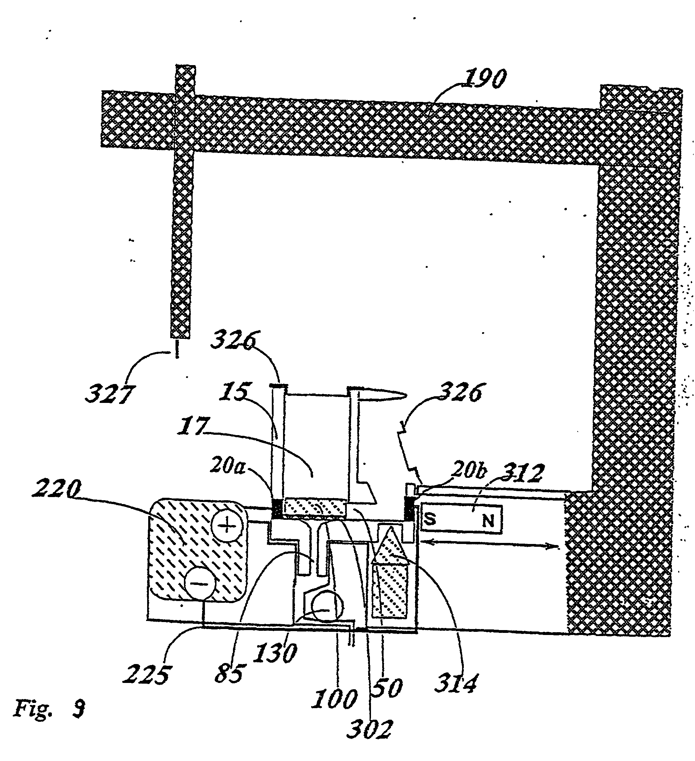

Fig. 9 zeigt in schematischer Querschnittsansicht ein zweites Ausführungsbeispiel einer Aufreinigungs- und Anreicherungsvorrichtung. Dabei sind die Elektroden 20a und 20b einer Vorrichtung gemäß Fig. 7 mit einer Spannungsquelle 220 verbunden. Die Vorrichtung gemäß Fig. 7 befindet sich im Wirkbereich eines x,y,z-Pipettierarms 190 eines x,y,z-Pipettierroboters. Die Spannungsquelle 220, die zweite Pumpe 130 zur Entsorgung von zu verwerfenden Lösungen, eine Vorrichtung (hier nicht gezeigt) zur Bewegung eines Permanentmagneten 312 sowie der x,y,z-Pipettierroboter sind mittels eines Prozeßrechners, bsp. eines Personal-Computers, vollautomatisch steuerbar.

Die Funktion der beschriebenen Vorrichtungen ist die folgende:

Zu analysierende biologische nukleinsäurehaltige Flüssigkeiten werden mit einem Adsorptionsmittel 100 in Kontakt gebracht. Dabei adsorbieren die in der Lösung befindlichen Nukleinsäuren am Adsorptionsmittel 100. Das mit den Nukleinsäuren beladene Adsorptionsmittel 100, bsp. ein Spin-column 90, wird durch die zweite Öffnung 80 in den Reaktionsraum 17 eingesetzt. Anschließend wird an die Elektroden 20a, 20b eine Gleichspannung im Bereich von 1 bis 5000 V, vorzugsweise von 25 bis 500 V, angelegt. Die negativ geladenen Nukleinsäuren werden dadurch vom Adsorptionsmittel 100 gelöst und in Richtung der in der Nähe des Entnahmeraums 50 angeordneten Anode 20b bewegt. Um einen direkten Kontakt der Nukleinsäuren mit der Anode 20b zu vermeiden, ist eine fur Nukleinsäuren undurchlässige erste permeable Membran 30 vorgesehen. Infolge der in Richtung der Anode 20b gerichteten Bewegung der Nukleinsäuren reichern sich diese im Entnahmeraum 50 an. Nach einer Elektrophoresedauer von 1 bis 180 min. wird der durch den Elektrophoresepuffer geleitete Strom abgeschaltet. Durch die Entnahmeöffnung 60 des Entnahmeraums 50 kann nun ein angereicherte Nukleinsäuren enthaltendes Elutionsvolumen entnommen werden.

Um Kontaminationen zu vermeiden, kann die Entnahmeöffnung 60 mit dem Schnappdeckel 326 verschlossen werden, der mit einem Septum 328 versehen ist. Zur Entnahme von Elutionsvolumen kann das Septum 328 mit einer Nadel 327 durchstochen werden.

Je nach Art der zu isolierenden Nukleinsäuren können verschiedenartig gestaltete Elektroden 20a, 20b verwendet werden. In Frage kommt die Verwendung von aus Edelmetall oder aus leitfähigen Kunststoffen hergestellten Elektroden, die beschichtet sein können.

Die erfindungsgemäße Vorrichtung kann mit einer Einrichtung zur Detektion der Chemilumineszenz kombiniert werden. Dazu ist ein Photomultiplier 314 in der Nähe des Behälters 15 angeordnet. Zunächst werden die Nukleinsäuren elektrophoretisch vom Adsorptionsmittel 100 in Richtung der Anode 20b bewegt. Im Bereich der Anode 20b kann sodann, bsp. nach der Polymerasekettenreaktion, eine Amplifikation der Nukleinsäuren durchgeführt werden. Die amplifizierten Nukleinsäuren werden dann durch Zugabe von Magnetpartikeln gebunden. Durch Heranführen des Permanentmagneten 312 an die Anode 20b werden die mit Nukleinsäuren beladenen Magnetpartikel an die Anode 20b angezogen. Nach Zugabe eines Chemilumineszenspuffers und Anlegen einer Spannung über den Elektroden 20a, 20b wird eine Chemilumineszens ausgelöst. Das dabei ausgesendete Licht wird durch den Photomultiplier 314 detektiert.

Die vorerwähnten Funktionen können mittels eines x,y,z-Pipettierroboters automatisiert werden. Damit ist es möglich, eine Mehrzahl der erfindungsgemäßen Vorrichtungen nacheinander automatisch zu bedienen.

Eine automatische Isolation von Nukleinsäuren kann mit den in den Fig. 4 und 9 gezeigten Aufreinigungs- und Anreicherungsvorrichtungen durchgeführt werden. Dabei hat sich folgendes Steuerungsprogramm als zweckmäßig erwiesen:

| Schritt Nr. | Gerätemodul | Arbeitsschritt |
|---|---|---|
| 1 | x,y,z-Pipettor | hole Pipettenspitze aus Vorrat 185 |
| 2 | x,y,z-Pipettor | gehe zu Probengefäß 165 |
| 3 | x,y,z-Pipettor | nehme 210 µl Probe auf |
| 4 | x,y,z-Pipettor | gehe zu Reaktionsröhrchen 150 |
| 5 | x,y,z-Pipettor | dispensiere 200 µl |
| 6 | x,y,z-Pipettor | verwerfe Pipettenspitze |
| 7 | x,y,z-Pipettor | hole Pipettenspitze aus Vorrat 185 |
| 8 | x,y,z-Pipettor | gehe zu erstem Gefäß 160 |
| 9 | x,y,z-Pipettor | nehme 710 µl Lyse-Reagenz auf |
| 10 | x,y,z-Pipettor | gehe zu Reaktionsröhrchen 150 |
| 11 | x,y,z-Pipettor | dispensiere 700 µl Lyse-Reagenz |
| 12 | x,y,z-Pipettor | verwerfe Pipettenspitze |
| 13 | Thermomixer | schüttle für 1 min |
| 14 | Thermomixer | erhitze auf 75 °C |
| 15 | Steuerung | warte 10 min. |
| 16 | Thermomixer | kühle auf 25 °C |
| 17 | x,y,z-Pipettor | hole Pipettenspitze aus Vorrat 185 |
| 18 | x,y,z,-Pipettor | gehe zu Reaktionsröhrchen 150 |
| 19 | x,y,z-Pipettor | nehme 810 µl Lysemischung auf |
| 20 | x,y,z-Pipettor | gehe zu zweiter Öffnung 80 |
| 21 | x,y,z-Pipettor | dispensiere 800 µl Lysemischung |
| 22 | x,y,z-Pipettor | verwerfe Pipettenspitze |
| 23 | Pumpe (130) | pumpe Lysemischung durch Adsorbtionsmittel und verwerfe |
| 24 | x,y,z-Pipettor | hole Pipettenspitze aus Vorrat 185 |
| 25 | x,y,z-Pipettor | gehe zu zweitem Gefäß 162 |
| 26 | x,y,z-Pipettor | nehme 710 µl Wasch-Reagenz auf |
| 27 | x,y,z-Pipettor | gehe zu zweiter Öffnung 80 |
| 28 | x,y,z-Pipettor | dispensiere 700 µl Waschlösung |
| 29 | x,y,z-Pipettor | verwerfe Pipettenspitze |
| 30 | zweite Pumpe 130 | pumpe Waschlösung durch Adsorbtionsmittel und verwerfe |
| 31 | erste Pumpe 120 | pumpe Elektrophoresepuffer in den Tank |
| 32 | x,y,z-Pipettor | hole Pipettenspitze aus Vorrat 185 |
| 33 | x,y,z-Pipettor | gehe zu Elektrophoresepuffervorrat 110 |
| 34 | x,y,z-Pipettor | nehme 250 µl Elektrophoresepuffer auf |
| 35 | x,y,z-Pipettor | gehe zu zweiter Öffnung 80 |
| 36 | x,y,z-Pipettor | verwerfe Pipettenspitze |
| 37 | Spannungsversorgung 220 | lege Spannung an Elektroden 20a, 20b |
| 38 | Steuerung | warte 20 min |
| 39 | x,y,z-Pipettor | hole Pipettenspitze aus Vorrat 185 |
| 40 | x,y,z-Pipettor | nehme 60 µl isolierte Nukleinsäure aus Entnahmeöffnung 60 auf |
| 41 | x,y,z-Pipettor | gehe zu PCR-Gefäßen 210 |
| 42 | x,y,z-Pipettor | dispensiere 50 µl in PCR-Gefäße 210 |
| 43 | x,y,z-Pipettor | verwerfe Pipettenspitze |
| 44 | zweite Pumpe 130 | pumpe Elektrophoresepuffer aus dem Tank und verwerfe |

### Beispiel 1

### Aufarbeitung einer Vollblutprobe mit Spin-column und Elektrophorese:

Alle Reagenzien sind aus dem QIAamp™Blood Kit (Kat.Nr. 29104) der Firma Qiagen, Hilden entnommen. Nach Lyse und Adsorbtion der Nukleinsäure laut Protokoll des Herstellers wurde das Glasfaservlies aus dem QIAamp spin column herausgenommen und in ein speziell präpariertes Agaroseflachbettgel gemäß Fig. 5 gegeben. Die erste Ausnehmung 81 dient zur Aufnahme des Glasvlieses und die zweite Ausnehmung 61 ist mit Elektrophorespuffer befüllt. Auf diese Weise kann die Nukleinsäure elektrophoretisch aus dem Glasvlies eluiert und in das anschließende Agarosegel und in die zweite Ausnehmung 61 überführt werden. Aus der zweiten Ausnehmung 61 wurde die isolierte konzentrierte Nukleinsäure entnommen.

### Beispiel 2

### Aufarbeitung einer Plasmaprobe

Alle Reagenzien sind aus dem QIAamp™Blood Kit (Kat.Nr. 29104) der Firma Qiagen, Hilden entnommen. Zur Aufarbeitung wurde das Glasfaservlies aus dem QIAamp spin column entfernt und in die Vorrichtung gemäß Fig.1 so eingesetzt, daß es am unteren Auslaß positioniert war. Das Volumen des gesamten Reaktionsgefäßes war 2 ml. Es wurden 200 µl Plasma laut Arbeitsanweisung des Herstellers verarbeitet. Statt Zentrifugation erfolgte das Absaugen mit einer Membranpumpe der Fa. Eppendorf. Zur elektrophoretischen Elution wurde ein Elektrophoresepuffer nach Andrews A.T. (Andrew A.T.: Electrophoresis, Claredon Press, Oxford, 1985, S. 160) verwendet. Als permeable Membran diente ein Dialyseschlauch der Fa. Neolab, Heidelberg (Best.Nr.: 2-9022). Als Elektroden 20a, 20b wurden Drähte mit 0,3 mm Durchmesser einer Platin-Ruthenium-Legierung verwendet und als Spannungsgeber der Elektrophoresespannungsgeber der Fa. Hölzel, Dorfen. Aus der Entnahmeöffnung 60 wurden 30 µl Elutionsvolumen mit der Nukleinsäure entnommen.

### Beispiel 3

### Isolierung von DNA aus Hühnerblut

Von einem frisch geschlachteten weißen Masthuhn wurde Vollblut aus der Halsschlagader aufgefangen und sofort mit Ethylendiamintetraessigsäure (Fa. Sigma, München Best. Nr. E-5513) in einer Konzentration von 0,06 g EDTA/ml Vollblut versetzt. Das EDTA-Vollblut wurde portioniert eingefroren und bei -15°C gelagert. Alle Reagenzien sind dem "High Pure PCR Template Preparation Kit" der Fa. Boehringer Mannheim (Best. Nr. 1 796 828) entnommen. 100 µl EDTA-Vollblut (s.o.) wurden mit 200 µl Lyse-Puffer und 60 µl Proteinase K jeweils aus dem o.g. Reagenziensatz gemischt und 15 min. bei 70°C inkubiert. Nach Abkühlen auf Raumtemperatur werden 100 µl Isopropanol (Fa. Roth, Karlsruhe Best.Nr. 9866) hinzugegeben und kräftig gemischt. Anschließend wird die zähflüssige Reaktionsmischung mit einer Vakuumpumpe (Eppendorf, Hamburg Nr. 4151) durch das Glasvlies gesaugt. Danach wurde das Vlies mit fünfmal 500 µl Waschpuffer (aus Kit s.o.) mit 80 % Ethanol (Fa. Roth, Karlsruhe Best. Nr. 5054) gewaschen. Dann wurde das Vlies aus dem Filter-Tube entfernt und in eine Vorrichtung gemäß Fig.5 in die erste Ausnehnung 81 überführt. Anschließend wurden ca. 0,5 ml Elektrophoresepuffer (10 mM Tris-HCl [Fa. Sigma, München Best.Nr. T-8529] 5 mM Natrium-Acetat [Fa Sigma, München Best. Nr. S-3272] 0,5 mM EDTA [s.o.] pH 8,2) auf das Vlies in die erste Ausnehmung 81 zupipettiert, der zuvor auf 70 °C erhitzt worden war. Danach wurde die Elektroelution durch Anlegen einer Gleichspannung von max. 10 mA bei ca. 60 grd. C durchgeführt. Als Spannungsquelle diente ein Elektrophoresetransformator der Fa Hölzel, Dorfen (Nr. 0 628/ 1985).Die Elution erfolgte in Fraktionen 1-7, wobei nach definierter Zeit (10-15 min) mit einer Eppendorfpipette Fraktionen von ca 50 µl aus der Öffnung (61) entnommen und gesammelt wurden. Die Fraktionen wurden auf einem Agarosegel (0,05 mg Agarose in 60 ml Elektrophoresepuffer mit 40 µl Ethidiumbromidlösung [100 mg Ethidiumbromid (Fa. Sigma, München Nr. E-8751) in dest. Wasser] analysiert. Als Kontrolle wurden 40 µl Lysemischung verwendet. Die Kontrolle und Fraktionen nach 15 min. Elektroelution zeigten eine fluoreszierende Bande nach Elektrophorese von 5 min. bei ca. 40 V und max. 50 mA unter Beleuchtung mit einer UV-Lampe der Fa. Roger Electronic Products (Nr. MD-1782GS). Als Spannungsquelle diente ein Elektrophoresetransformator der Fa Hölzel, Dorfen (Nr. 0 628/ 1985).

### Beispiel 5

### Herstellung einer beschichteten elektrisch leitfähigen Kunststoffelektrode (Fig. 6)

Zunächst wird biotinyliertem Rinder-Immunglobulin G (R-IgG) hergestellt. Dazu werden 0,5 ml einer R-IgG-Lösung (2mg R-IgG (Boehringer Mannheim Cat.No. 1293621103 in 1 ml PBS ( NaH2PO4*1 H2O 2,76 g/l; Na2HPO4*2H2O 3,56 g/l; NaCl 8 g/l; pH 7,25)) mit 6 µl D-Biotinoyl-ε-aminocapronsäure-N-hydroxysuccinimidesterlösung in PBS und DMSO (Ansatz laut Biotin Labeling Kit von Boehringer Mannheim Best. Nr. 1418165) vermischt und 2,5 h bei Raumtemperatur auf einem Magnetrührer gerührt und anschließend über Nacht stehen lassen. Das molare Verhältnis von Biotin: R-IgG beträgt 20:1 bei diesem Ansatz.

Zur Beschichtung von elektrisch leitfähigem Kunststoff mit biotinyliertem R-IgG werden aus einem Rohling, hergestellt im Spritzgußverfahren aus PRE-ELEC TP 4474 (Premix Oy, Finnland), Scheiben von 4 mm Durchmesser ausgeschnitten, in einen Napf einer unbeschichteten Mikrotiterplatte gelegt und in einer Lösung von 0,2 ml Beschichtungspuffer (NaHCO3 4,2 g/l; pH 9,6) dreimal, dann in einer Lösung von 40 ml Beschichtungspuffer (NaHCO3 4,2 g/l;pH 9,6) und 6 µl R-IgG-Biotin-Lösung gewaschen. Die Beschichtung erfolgt über Nacht.

Anschließend werden die Scheiben 3 x mit je 100 ml Milli-Q-Wasser gewaschen, wobei durch Sedimentation oder Zentrifugatiön die Trennung von fester und flüssiger Phase erfolgt. Anschließend werden die Scheiben in 40 ml PBS wieder aufgenommen.

### Beispiel 6

### Durchführung einer Probenvorbereitung mit Elektroelution, Amplifikation und Elektrochemilumineszenz-Messung zum Nachweis von PCR-Amplifikaten

Die in Fig. 9 gezeigte Vorrichtung wurde zur Durchführung der Isolierung, Amplifikation und Chemilumineszenzmessung automatisch mit einem Computer-Programm mit den folgenden Programmschritten gesteuert:

| **Prozeßmodule** | **Einzelschritte** |
|---|---|
| **Probenvorbereitung** | |
| Lyse | Probe, Lysemischung, Proteinase K in Reaktionsraum 17 pipettieren |
| | Reaktionsraum 17 verschließen |
| | Reaktionsraum 17 auf 70 °C temperieren |
| | Reaktionsraum 17 auf RT kühlen |
| | Reaktionsraum 17 öffnen |
| | Isopropanol zugeben |
| | Reaktionsmischung aus Reaktionsraum 17 durch zweiten Stutzen 85 absaugen |
| Elektroelution | Elutionspuffer zugeben |
| | Spannung an Elektroden 20a, 20b anlegen |
| | Nukleinsäure wandert in den Entnahmeraum 50 |
| **Amplifikation** | Zugabe von PCR-Mix in den Entnahmeraum 50 |
| | Verschließen von zweitem Stutzen 85, zweiter Öffnung 80 und Entnahmeöffnung 60 |
| | zyklisches Heizen und Abkühlen des Entnahmeraums 50 |
| **Denaturierung** | Öffnen der Entnahmeöffnung 60 |
| Sonden annealing | Zugabe von Ru-Sonde |
| | Verschließen von Entnahmeöffnung 60 |
| | Aufheizen und Abkühlen des Entnahmeraums 50 |
| **Detektion** | Öffnen von Entnahmeöffnung 60 |
| | Zugabe von SA-Magnetpartikel durch Entnahmeöffnung 60 |
| | Verschließen von Entnahmeöffnung 60 |
| Magnetseparation | Anlegen von Dauermagnet 312 mit Magnetfeld für den Entnahmeraum 50 |
| | Absaugen von Reaktionsmischung aus dem Entnahmeraum 50 |
| Magnetpartikel waschen (Option) | Zugabe von Waschlösung durch Entnahmeöffnung 60 |
| | Entfemen des Magnetfeldes für den Entnahmeraum 50 |
| | Absaugen der Waschlösung durch zweiten Stutzen 85 |
| | Zugabe von Assay-puffer durch Entnahmeöffnung 60 |
| Elektrochemilumineszenz-Messung | Anlegen von Spannung an die Elektroden 20a,b |
| | Messung der Lumineszens mittels Photomultiplier 314 |

### Bezugszeichenliste

- 10: Elektrophorosepuffertank
- 11: Agaroseflachbettgel
- 15: Behälter
- 17: Reaktionsraum
- 20a: Kathode
- 20b: Anode
- 30: erste permeable Membran
- 31: zweite permeable Membran
- 40: 0-Ring
- 41: erster Stutzen
- 42: erste Öffnung
- 43: Durchbruch
- 44: zweiter Stutzen
- 45: dritter Stutzen
- 46: dritte Öffnung
- 49: Kanal
- 50: Entnahmeraum
- 60: Entnahmeöffnung
- 61: zweite Ausnehmung
- 70: Füllstand
- 80: zweite Öffnung
- 81: erste Ausnehmung
- 85: zweiter Stutzen
- 90: Spin-column
- 100: Adsorptionsmittel
- 110: Elektrophoresepuffervorrat
- 120: erste Pumpe
- 125: Füllstutzen
- 130: zweite Pumpe
- 140: Ableitung
- 150: Reaktionsröhrchen
- 160: erstes Gefäß
- 162: zweites Gefäß
- 165: Probengefäß
- 170: Schüttelbock
- 180: Pipettenspitze
- 185: Vorrat an Pipettenspitzen
- 190: x,y,z-Pipettierarm
- 210: PCR-Gefäße
- 220: Spannungsquelle
- 225a, b: elektrische Zuleitungen
- 302: Stützvlies
- 303: weiteres Stützvlies
- 310: semipermeable Membran
- 312: Permanentmagnet
- 313: Magnetpartikel
- 314: Photomultiplier
- 316: durchsichtiger Schnappdeckel
- 318: Schlauchstück
- 320, 321: Ausnehmungen
- 323: erste Lage
- 324: dritte Lage
- 325: zweite Lage
- 326: Schnappdeckel
- 327: Nadel
- 328: Septum
- 329: Heizplatten
- 340: Stopfen
- 400: Deckel
- 401: Vakuumanschluß
- 402: Anschlüsse
- 410: Mehrfachbehälter

## Patentansprüche

1. Verfahren zur kontaminationsfreien Isolierung von Nukleinsäuren aus biologischen nukleinsäurehaltigen Flüssigkeiten und Suspensionen, wobei
a) die Nukleinsäuren an ein in einem Reaktionsraum (17) aufgenommenes Adsorptionsmittel (100) gebunden werden,
b) die Nukleinsäuren vom Adsorptionsmittel (100) eluiert und
c) durch Elektrophorese vom Reaktionsraum (17) uber einen Kanal (49) in einen damit verbundenen Entnahmeraum (50) bewegt und dort angereichert werden.

2. Verfahren nach Anspruch 1, wobei das Adsorptionsmittel (100) nach der Adsorption gewaschen wird.

3. Verfahren nach Anspruch 1 oder 2, wobei die Elution der Nukleinsäuren vom Adsorptionsmittel (100) mittels Pufferwechsel oder durch Elektrophorese erfolgt.

4. Verfahren nach einem der Anspruche 1 bis 3, wobei das im Entnahmeraum (50) befindliche Elutionsvolumen im Bereich von 0,005 bis 0,1ml liegt und kleiner ist als das Probenausgangsvolumen.

5. Verfahren nach einem der Ansprüche 1 bis 4, wobei die Nukleinsauren vor der Adsorption durch Lyse freigesetzt werden.

6. Verfahren nach einem der Ansprüche 1 bis 5, wobei biologischen Flüssigkeiten oder Suspensionen durch Verflüssigung aus festem Material hergestellt werden.

7. Verfahren nach einem der Ansprüche 1 bis 6, wobei das Adsorptionsmittel (100) aus Silicagel, Glaspartikeln, Glasfaservlies oder Ionenaustauschmaterial besteht.

8. Verfahren nach einem der vorhergehenden Ansprüche, wobei das Adsorptionsmittel (100) aus mit Glas umfangenen magnetischen Partikeln besteht, und die an magnetischen Partikel adsorbierten Nukleinsauren mittels eines Magneten (312) isoliert werden.

9. Verfahren nach einem der vorhergehenden Anspruche, wobei nach der Elution eine Hybridisierung durchgeführt wird.

10. Verfahren nach einem der vorhergehenden Anspruche, wobei nach der Elution eine Amplifikation durchgeführt wird.

11. Verfahren nach einem der vorhergehenden Anspruche, wobei nach der Elution eine Chemilumineszenzdetektion durchgefuhrt wird.

12. Vorrichtung zur kontaminationsfreien Isolierung von Nukleinsäuren aus biologischen nukleinsäurehaltigen Flüssigkeiten und Suspensionen, wobei ein Reaktionsraum (17) zur Aufnahme eines mit Nukleinsäuren beladenen Absorptionsmittels (100) über einen Kanal (49) mit einem Entnahmeraum (50) verbunden ist, und wobei die Nukleinsauren mittels einer Elektrophoreseeinrichtung (20a, 20b) vom Reaktions- (17) in den Entnahmeraum (50) bewegbar und dort anreicherbar sind.

13. Vorrichtung nach Anspruch 12, wobei der Reaktionsraum (17) von einem Elektrophoresepuffertank (10) umgeben ist.

14. Vorrichtung nach Anspruch 13, wobei der Reaktionsraum (17) mit dem Elektrophoresepuffertank (10) ionenleitend verbunden ist.

15. Vorrichtung nach Anspruch 13 oder 14, wobei der Reaktionsraum (17) sich im Elektrophoresepuffertank (10) befindet.

16. Vorrichtung nach einem der Ansprüche 12 bis 15, wobei der Reaktionsraum (17) über mindestens eine Offnung (80) beschickbar und entsorgbar ist.

17. Vorrichtung nach einem der Anspruche 12 bis 16, wcbei im Reaktionsraum (17) ein Adsorptionsmittel (100) für Nukleinsäuren aufgenommen ist.

18. Vorrichtung nach einem der Anspruche 12 bis 17, wobei die lonenleitende Verbindung durch mindestens eine permeable, Nukleinsauren zuruckhaltende Membran (31) gebildet ist.

19. Vorrichtung nach einem der Ansprüche 12 bis 16, wobei die Vorrichtung aus thermoplastischem Kunststoff hergestellt ist.

20. Vorrichtung nach einem der Ansprüche 12 bis 19, wobei die Elektrophoreseeinrichtung mindestens zwei Elektroden (20a, 20b) aufweist, von denen mindestens eine in den Elektrophoresepuffertank (10) ragt oder Bestandteil desselben ist.

21. Vorrichtung nach einem der Ansprüche 12 bis 19, wobei die Elektrophoreseeinrichtung mindestens zwei Elektroden (20a, 20b) aufweist, von denen mindestens eine in den Entnahmeraum (50) ragt oder Bestandteil eines den Entnahmeraum (50) umgreifenden Behälters (15) ist.

22. Vorrichtung nach Anspruch 20 oder 21, wobei eine der Elektroden (20a, 20b) in den Reaktionsraum (17) ragt oder Bestandteil eines den Reaktionsraum (17) umgreifenden Behälters (15) ist.

23. Vorrichtung nach einem der Ansprüche 20 bis 22, wobei mindestens eine der Elektroden (20a, 20b) aus einem elektrisch leitfähigen Kunststoff hergestellt ist.

24. Vorrichtung nach Anspruch 23, wobei der elektrisch leitfähige Kunststoff elektrisch leitfahige Zuschlagstoffe, wie Graphit, Eisen, Silber oder andere Metalle, enthält.

25. Vorrichtung nach einem der Ansprüche 20 bis 24, wobei der Widerstand der Elektroden (20a, 20b) kleiner als 100 MΩ ist.

26. Vorrichtung nach einem der Anspruche 20 bis 25, wobei die Elektroden (20a, 20b) eine Beschichtung aufweisen.

27. Vorrichtung nach Anspruch 26, wobei die Beschichtung der Elektroden (20a, 20b) aus mehreren Lagen (323, 324, 325) besteht.

28. Vorrichtung nach Anspruch 27, wobei mindestens eine Lage (323, 324, 325) aus einem bindefähigen Protein gebildet ist.

29. Vorrichtung nach Anspruch 28, wobei das bindefahige Protein ein Antikorper, ein Antigen oder eine Komponente eines anderen Ligand-Rezeptor-Paares ist.

30. Vorrichtung nach Anspruch 28, wobei das biologische Polymer derart ist, daß daran Nukleinsäuren bindbar sind.

31. Vorrichtung nach Anspruch 28, wobei das biologische Polymer ein Oligonukleotid ist.

32. Vorrichtung nach Anspruch 28, wobei das biologische Polymer ein proteinähnliches Aminosäure-Konstrukt (PNA-Konstrukt) ist.

33. Vorrichtung nach Anspruch 27, wobei eine der Lagen (323, 324, 325) aus chemisch reaktiven Linkermolekülen besteht.

34. Vorrichtung nach Anspruch 20 oder 21, wobei zwei Elektroden (20a, 20b) in Gegenüberstellung in der Wandung des Behälters (15) vorgesehen sind.

35. Vorrichtung nach einem der Ansprüche 12 bis 34, wobei Mittel (312) zu Erzeugung eines an- und abschaltbaren Magnetfelds so angeordnet sind, daß der Behälter (15) vom Magnetfeld durchdringbar sind.

36. Vorrichtung nach einem der Ansprüche 12 bis 35, wobei in der Nähe des Behälters (15) eine Detektionseinrichtung, vorzugsweise ein Photomultiplier (314), angeordnet ist.

37. Vorrichtung nach Anspruch 36, wobei der Behalter (15) ein optisches Fenster (316) besitzt.

38. Elutionsvorrichtung mit einer Mehrzahl von Vorrichtungen nach einem der Ansprüche 12 bis 37, wobei die den Vorrichtungen zugeordneten Elektroden (20a, 20b) elektrisch miteinander und mit einer Stromquelle (220) verbunden sind.

39. Elutionsvorrichtung nach Anspruch 38, wobei die Vorrichtungen geometrisch im 96-Napf-Mikrotitrationsplattenformat angeordnet sind.

40. Aufreinigungs- und Anreicherungsvorichtung mit einer Vorrichtung nach einem der Anspruche 12 bis 37 oder einer Elutionsvorrichtung nach Anspruch 38 oder 39, wobei mindestens eine der folgenden Einrichtungen vorgesehen ist:
- x,y,z-Pipettierarm (190),
- Thermo-Schuttelbock (170),
- Heiz-/Kühlmittel (329) zum zyklischen Heizen und Kühlen,
- Spannungsquelle (220),
- mindestens zwei Elektroden (20a, 20b),
- mindestens eine Pumpe (120, 130),
- mindestens ein Magnet (312),
- mindestens ein Photomultiplier (314).

41. Aufreinigungs- und Anreicherungsvorichtung nach Anspruch 40, wobei die Vorrichtung, die Elutionsvorrichtung und die Einrichtung/en mittels eines Prozeßrechners automatischen zur Durchfuhrung des Verfahrens nach einem der Anspruche 1 bis 11 steuerbar sind.

## Claims

1. Process for the contamination-free isolation of nucleic acids from biological fluids and suspensions containing nucleic acids,
a) the nucleic acids being bound to an adsorption medium (100) held in a reaction compartment (17),
b) the nucleic. acids being eluted from the adsorption medium (100) and
c) being moved by electrophoresis from a reaction compartment (17) via a channel (49) into a removal compartment (50) connected thereto and enriched there.

2. Process according to claim 1, characterized in that the adsorption medium (100) is washed after the adsorption.

3. Process according to claim 1 or 2, characterized in that the nucleic acids are eluted from the adsorption medium (100) by buffer change or by electrophoresis.

4. Process according to one of claims 1 to 3, characterized in that the elution volume present in the removal compartment (50) is in the range from 0.005 to 0.1 ml and is smaller than the original sample volume.

5. Process according to one of claims 1 to 4, characterized in that the nucleic acids are released by lysis before the adsorption.

6. Process according to one of claims 1 to 5, characterized in that biological fluids or suspensions are prepared from solid material by liquefaction.

7. Process according to one of claims 1 to 6, characterized in that the adsorption medium (100) consists of silica gel, glass particles, glass fiber fleece or ion exchange material.

8. Process according to one of the preceding claims, characterized in that the adsorption medium (100) consists of glass-encoated magnetic particles, and the nucleic acids adsorbed to magnetic particles are isolated by means of a magnet (312).

9. Process according to one of the preceding claims, characterized in that a hybridization is carried out after the elution.

10. Process according to one of the preceding claims, characterized in that an amplification is carried out after the elution.

11. Process according to one of the preceding claims, characterized in that a chemiluminescence detection is carried out after the elution.

12. Apparatus for the contamination-free isolation of nucleic acids from biological fluids and suspensions containing nucleic acids, characterized in that a reaction compartment (17) for holding an adsorption medium (100) laden with nucleic acids is connected via a channel (49) to a removal compartment (50), and characterized in that the nucleic acids can be moved by an electrophoresis device (20a, 20b) from the reaction compartment (17) into the removal compartment (50) and enriched there.

13. Apparatus according to claim 12, characterized in that the reaction compartment (17) is surrounded by an electrophoresis buffer tank (10).

14. Apparatus according to claim 13, characterized in that the reaction compartment (17) is connected to the electrophoresis buffer tank (10) in an ion-conducting manner.

15. Apparatus according to claim 13 or 14, characterized in that the reaction compartment (17) is situated in the electrophoresis buffer tank (10).

16. Apparatus according to one of claims 12 to 15, characterized in that the reaction compartment (17) can be charged and drained via at least one orifice (85).

17. Apparatus according to one of claims 12 to 16,
characterized in that an adsorption medium (100) for nucleic acids is held in the reaction compartment (17).

18. Apparatus according to one of claims 12 to 17, characterized in that the ion-conducting connection is formed by at least one permeable membrane (31) which retains nucleic acids.

19. Apparatus according to one of claims 12 to 18, characterized in that the apparatus is fabricated from thermoplastic.

20. Apparatus according to one of claims 12 to 19, characterized in that the electrophoresis device has at least two electrodes (20a, 20b) of which at least one projects into the electrophoresis buffer tank (10) or is a component of the same.

21. Apparatus according to one of claims 12 to 19,
characterized in that the electrophoresis device has at least two electrodes (20a, 20b), of which at least one projects into the removal compartment (50) or is a of a container (15) enclosing the removal compartment (50).

22. Apparatus according to claim 20 or 21,
characterized in that one of the electrodes (20a, 20b) projects into the reaction compartment (17) or is a component of the container (15) enclosing the reaction compartment (17).

23. Apparatus according to one claims 20 to 22, characterized in that at least one of the electrodes (20a, 20b) is fabricated from an electrically conducting plastic.

24. Apparatus according to claim 23, characterized in that the electrically conductive plastic comprises electrically conductive additives, such as graphite, iron, silver or other metals.

25. Apparatus according to one of claims 20 to 24, characterized in that the resistance of the electrodes (20a, 20b) is less than 100 MΩ.

26. Apparatus according to one of claims 20 to 25, characterized in that the electrodes (20a, 20b) have a coating.

27. Apparatus according to claim 26, characterized in that the coating of the electrodes (20a, 20b) consists of a plurality of layers (323, 324, 325).

28. Apparatus according to claim 27, characterized in that at least one layer (323, 324, 325) is formed from a protein capable of binding.

29. Apparatus according to claim 28, characterized in that the protein capable of binding is an antibody, an antigen or a component of another ligand-receptor pair.

30. Apparatus according to claim 28, characterized in that the biological polymer is of a type such that nucleic acids can bind thereto.

31. Apparatus according to claim 28, characterized in that the biological polymer is an oligonucleotide.

32. Apparatus according to claim 28, characterized in that the biological polymer is a protein-like amino acid construct (PNA construct).

33. Apparatus according to claim 27, characterized in that one of the layers (323, 324, 325) consists of chemically reactive linker molecules.

34. Apparatus according to claim 20 or 21, characterized in that two electrodes (20a, 20b) are provided opposite each other in the wall of the container (15).

35. Apparatus according to one of claims 12 to 34, characterized in that means (312) for producing a magnetic field which can be switched on and off are arranged in such a manner that the container (15) can be penetrated by the magnetic field.

36. Apparatus according to one of claims 12 to 35, characterized in that in the vicinity of the container (15) a detection device, preferably a photomultiplier (314), is arranged.

37. Apparatus according to claim 36, characterized in that the container (15) has an optical window (316).

38. Elution apparatus having a plurality of apparatuses according to one of claims 12 to 37,
characterized in that the electrodes (20a, 20b) assigned to the apparatuses are electrically connected to one another and to a supply (220).

39. Elution apparatus according to claim 38,
characterized in that the apparatuses are arranged geometrically in the 96-well microtitration plate format.

40. Purification and enrichment apparatus having an apparatus according to one of claims 12 to 37 or an elution apparatus according to claim 38 or 39, characterized in that at least one of the following devices is provided:
- x,y,z-pipetting arm (190)
- thermostated shaker frame (170)
- heating/cooling medium (329) for the cyclic heating and cooling,
- power supply (220),
- at least two electrodes (20a, 20b),
- at least one pump (120, 130),
- at least one magnet (312),
- at least one photomultiplier (314).

41. Purification and enrichment apparatus as claimed in claim 40, characterized in that the apparatus, the elution apparatus and the device(s) can be automatically controlled by means of a process computer for carrying out the process according to one of claims 1 to 11.

## Revendications

1. Procédé pour isoler sans contamination des acides nucléiques à partir de fluides et de suspensions biologiques contenant des acides nucléiques, dans lequel
a) les acides nucléiques sont liés à un moyen d'adsorption (100) logé dans une chambre de réaction (17),
b) les acides nucléiques sont élués du moyen d'adsorption (100), et
c) déplacés par électrophorèse de la chambre de réaction (17) dans une chambre de prélèvement qui y est reliée (50) par le biais d'un canal (49), et concentrés dans celle-ci.

2. Procédé selon la revendication 1, dans lequel le moyen d'adsorption (100) est lavé après l'adsorption.

3. Procédé selon la revendication 1 ou 2, dans lequel l'élution des acides nucléiques du moyen d'adsorption (100) se fait au moyen d'une modification de tampon ou par électrophorèse.

4. Procédé selon l'une des revendications 1 à 3, dans lequel le volume d'élution se trouvant dans la chambre de prélèvement (50) est compris dans la gamme allant de 0,005 à 0,1 ml, et est inférieur au volume de départ des échantillons.

5. Procédé selon l'une des revendications 1 à 4, dans lequel les acides nucléiques sont libérés par lyse avant l'adsorption.

6. Procédé selon l'une des revendications 1 à 5, dans lequel les fluides ou les suspensions biologiques sont préparés par liquéfaction à partir de matière solide.

7. Procédé selon l'une des revendications 1 à 6, dans lequel le moyen d'adsorption (100) se compose de gel de silice, de particules de verre, d'un non-tissé de fibres de verre ou d'un matériau échangeur d'ions.

8. Procédé selon l'une des revendications précédentes, dans lequel le moyen d'adsorption (100) se compose de particules magnétiques entourées de verre, et les acides nucléiques adsorbés sur les particules magnétiques sont isolés au moyen d'un aimant (312).

9. Procédé selon l'une des revendications précédentes, dans lequel on procède à une hybridation après l'élution.

10. Procédé selon l'une des revendications précédentes, dans lequel on procède à une amplification après l'élution.

11. Procédé selon l'une des revendications précédentes, dans lequel on procède à une détection de chimiluminescence après l'élution.

12. Dispositif pour isoler sans contamination des acides nucléiques à partir de fluides et de suspensions biologiques contenant des acides nucléiques, dans lequel une chambre de réaction (17), destinée à la réception d'un moyen d'adsorption (100) chargé en acides nucléiques, est reliée par un canal (49) à une chambre de prélèvement (50), et dans lequel les acides nucléiques peuvent être déplacés au moyen d'un dispositif d'électrophorèse (20a, 20b) de la chambre de réaction (17) dans la chambre de prélèvement (50), et y être concentrés.

13. Dispositif selon la revendication 12, dans lequel la chambre de réaction (17) est entourée par un réservoir tampon d'électrophorèse (10).

14. Dispositif selon la revendication 13, dans lequel la chambre de réaction (17) est reliée avec le réservoir tampon d'électrophorèse (10) par conduction d'ions.

15. Dispositif selon la revendication 13 ou 14, dans lequel la chambre de réaction (17) se trouve dans le réservoir tampon d'électrophorèse (10).

16. Dispositif selon l'une des revendications 12 à 15, dans lequel la chambre de réaction (17) peut être chargée et déchargée par au moins une ouverture (80).

17. Dispositif selon l'une des revendications 12 à 16, dans lequel un moyen d'adsorption (100) pour les acides nucléiques est logé dans la chambre de réaction (17).

18. Dispositif selon l'une des revendications 12 à 17, dans lequel la liaison par conduction d'ions est formée par au moins une membrane perméable de rétention des acides nucléiques (31).

19. Dispositif selon l'une des revendications 12 à 16, dans lequel le dispositif est fabriqué à partir d'une matière synthétique thermoplastique.

20. Dispositif selon l'une des revendications 12 à 19, dans lequel le dispositif d'électrophorèse présente au moins deux électrodes (20a, 20b), parmi lesquelles au moins une fait saillie dans le réservoir tampon d'électrophorèse (10) ou est une partie de celle-ci.

21. Dispositif selon l'une des revendications 12 à 19, dans lequel le dispositif d'électrophorèse présente au moins deux électrodes (20a, 20b), parmi lesquelles au moins une fait saillie dans la chambre de prélèvement (50) ou est une composante d'un réservoir (15) entourant la chambre de prélèvement (50).

22. Dispositif selon la revendication 20 ou 21, dans lequel une des électrodes (20a, 20b) fait saillie dans la chambre de réaction (17) ou est une composante d'un réservoir (15) entourant la chambre de réaction (17).

23. Dispositif selon l'une des revendications 20 à 22, dans lequel au moins une des électrodes (20a, 20b) est fabriquée à partir d'une matière synthétique électriquement conductrice.

24. Dispositif selon la revendication 23, dans lequel la matière électriquement conductrice contient des additifs électriquement conducteurs, comme le graphite, le fer, l'argent ou d'autres métaux.

25. Dispositif selon l'une des revendications 20 à 24, dans lequel la résistance des électrodes (20a, 20b) est inférieure à 100 MΩ.

26. Dispositif selon l'une des revendications 20 à 25, dans lequel les électrodes (20a, 20b) présentent un revêtement.

27. Dispositif selon la revendication 26, dans lequel le revêtement des électrodes (20a, 20b) se compose de plusieurs couches (323, 324, 325).

28. Dispositif selon la revendication 27, dans lequel au moins une couche (323, 324, 325) est formée d'une protéine apte à la liaison.

29. Dispositif selon la revendication 28, dans lequel la protéine apte à la liaison est un anticorps, un antigène ou une composante d'une autre paire ligand-récepteur.

30. Dispositif selon la revendication 28, dans lequel le polymère biologique est tel que des acides nucléiques peuvent s'y lier.

31. Dispositif selon la revendication 28, dans lequel le polymère biologique est un oligonucléotide.

32. Dispositif selon la revendication 28, dans lequel le polymère biologique est une construction d'acide aminé de type protéine (construction PNA).

33. Dispositif selon la revendication 27, dans lequel une des couches (323, 324, 325) se compose de molécules lieuses chimiquement réactives.

34. Dispositif selon la revendication 20 ou 21, dans lequel deux électrodes (20a, 20b) sont prévues en opposition dans la paroi du réservoir (15).

35. Dispositif selon l'une des revendications 12 à 34, dans lequel sont disposés des moyens (312) de génération d'un champ magnétique pouvant être commuté et non commuté de façon telle que le réservoir (15) peut être traversé par le champ magnétique.

36. Dispositif selon l'une des revendications 12 à 35, dans lequel est disposé, à proximité du réservoir (15), un dispositif de détection, de préférence un photomultiplicateur (314).

37. Dispositif selon la revendication 36, dans lequel le réservoir (15) possède une fenêtre optique (316).

38. Dispositif d'élution comportant plusieurs dispositifs selon l'une des revendications 12 à 37, dans lequel les électrodes (20a, 20b) coordonnées aux dispositifs sont reliées électriquement les unes aux autres, et avec une source de courant (220).

39. Dispositif d'élution selon la revendication 38, dans lequel les dispositifs sont disposés géométriquement dans un format de plaque de micro-titration à 96 puits.

40. Dispositif de purification et de concentration comportant un dispositif selon l'une des revendications 12 à 37 ou un dispositif d'élution selon la revendication 38 ou 39, dans lequel est prévu au moins un des appareils suivants :
- un bras de pipettage x,y,z (190),
- un support d'agitateur thermique (170),
- un élément de chauffage/refroidissement (329) permettant un chauffage et un refroidissement cycliques,
- une source de tension (320),
- au moins deux électrodes (20a, 20b),
- au moins une pompe (120, 130),
- au moins un aimant (312),
- au moins un photomultiplicateur (314).

41. Dispositif de purification et de concentration selon la revendication 40, dans lequel le dispositif, le dispositif d'élution et le ou les appareils peuvent être commandés automatiquement au moyen d'un ordinateur industriel pour exécuter le procédé selon l'une des revendications 1 à 11.
